(19) Europäisches Patentamt

European Patent Office

Office européen des brevets

(11) **EP 1 228 774 B1**

(12) **EUROPEAN PATENT SPECIFICATION**

(45) Date of publication and mention
of the grant of the patent:
**20.04.2005 Bulletin 2005/16**

(21) Application number: **01954464.2**

(22) Date of filing: **06.08.2001**

(51) Int Cl.⁷: $A61L\ 27/10$, $A61F\ 2/30$

(86) International application number:
**PCT/JP2001/006744**

(87) International publication number:
**WO 2002/011780 (14.02.2002 Gazette 2002/07)**

(54) **ARTIFICIAL JOINT MADE FROM ZIRCONIA-ALUMINA COMPOSITE CERAMIC**

KÜNSTLICHES GELENK AUS EINER ZIRKONIUM-ALUMINIUMVERBUNDKERAMIK

JOINT ARTIFICIEL EN CERAMIQUE COMPOSITE DE ZIRCONE-ALUMINE

(84) Designated Contracting States:
**DE FR GB IT**

(30) Priority: **07.08.2000 JP 2000239209**

(43) Date of publication of application:
**07.08.2002 Bulletin 2002/32**

(73) Proprietors:
• **Matsushita Electric Works, Ltd.
Kadoma-shi, Osaka-fu 571-8686 (JP)**
• **Kobe Steel, Ltd.
Kobe-shi, Hyogo 651-8585 (JP)**
• **Nakamura, Takashi
Kyoto 615-0005 (JP)**
• **KOKUBO, Tadashi
Nagaokakyo-shi, Kyoto 617-0841 (JP)**

(72) Inventors:
• **NAWA, Masahiro,
MATSUSHITA ELECTRIC WORKS LTD.
Kadoma-shi, Osaka 571-8686 (JP)**

• **MATSUSHITA, Tomiharu, c/o KOBE STEEL LTD.
Chuo-ku, Kobe-shi, Hyogo 651-0072 (JP)**
• **KANAMARU, Moriyoshi, c/o KOBE STEEL LTD.
Kobe-shi, Hyogo 651-2271 (JP)**
• **NAKAMURA, Takashi
Kyoto-shi, Kyoto 615-0005 (JP)**
• **The other inventors have agreed to waive their
entitlement to designation.**

(74) Representative: **Appelt, Christian W.
FORRESTER & BOEHMERT
Anwaltssozietät
Pettenkoferstrasse 20-22
80336 München (DE)**

(56) References cited:
| | |
|---|---|
| EP-A- 0 435 677 | EP-A- 0 834 484 |
| WO-A-99/27871 | DE-A- 19 540 452 |
| JP-A- 11 056 884 | JP-A- 11 228 221 |
| US-A- 4 690 911 | |

**Description**

TECHNICAL FILED

The present invention relates to an artificial joint made from a zirconia-alumina composite ceramic, which has excellent wear resistance and high mechanical strength, and provides good joint motion and function for a long time period with a high degree of reliability.

BACKGROUND ART

In the past, artificial joints have been used to restore the joint motion and function of patients with damaged joints or arthritic joints. For example, a conventional artificial hip joint is composed of an acetabular cup of an ultra-high molecular weight polyethylene and a bone member of a cobalt-chromium alloy or a ceramic such as alumina or zirconia. The bone member has a caput of a substantially spherical shape, which is slidably engaged to an inner surface of the acetabular cup to provide a joint motion of the artificial hip joint. When the combination of polyethylene and the metal or ceramic material is adopted, the wear amount of polyethylene is much larger than that of the metal or ceramic material. In recent years, it is reported that the use of ceramic material, and particularly zirconia is effective to reduce the wear amount of polyethylene.

On the other hand, when the artificial joint is used for a long time period, fine wear debris are generated from the artificial joint. For example, when large amounts of wear debris of polyethylene are generated, it causes a serious problem described below. That is, since the wear debris of polyethylene are of fine powders with submicron to micron particle size, they become a cause of Osteolysis that is dissolution phenomena of bones induced around the artificial joint in vivo. In addition, loosening is caused at the artificial joint by the abrasion loss of polyethylene. As a result, it is needed to replace the used artificial hip joint by a new one after about 10 to 15 years from the initial medical operation.

As a conventional artificial joint, for example, US Patent No. 6,241,773 discloses a biomedical article made from an alumina ceramic. This alumina ceramic contains alumina having a purity of 99.95% or more and 100 ppm or less of calcia or magnesia. This alumina ceramic has excellent wear resistance and a high three-point bending strength of 600 MPa or more. However, there is a common view that variations in mechanical properties of alumina ceramics are relatively large. Therefore, from the viewpoints of providing a stable joint motion with a high degree of reliability for a long time period, there is still plenty of room for improvement. In addition, there is a problem that it is difficult to stably supply the above-mentioned alumina ceramic with improved production yields.

On the other hand, the inventors of the present application proposed a zirconia-alumina composite ceramic sintered body for a biological use in Japanese Patent [Early] Publication No. 11-228221. This ceramic sintered body is composed of a first phase of zirconia grains containing ceria and titania as stabilizers for tetragonal zirconia, and a second phase of alumina grains. This ceramic sintered body is of a porous structure, and has an elastic modulus of 150 GPa and a high bending strength (170 MPa) as compared with conventional Apatite. However, to provide an increased wear resistance and a smooth joint motion in the use of the artificial joint in vivo for the long time period, there is plenty of room for further improvement of the conventional artificial joint.

Document WO 99/27871 describes artificial joints of prostheses, wherein a sintered material is provided, which is comprised of zircon oxide with an addition of 0,1 to 40 wt. % aluminum oxide.

Document EP 0 435 677 A2 describes an alumina-zirconia composite sintered product which comprises a zirconia crystalline phase composed mainly of tetragonal zirconia and an alumina crystalline phase.

Document EP 0 834 484 A 1 describes a zirconium dioxid based ceramic material and a method of producing the same. The material comprises a first phase of $ZrO_2$ grains containing $CeO_2$ as a stabilizer.

From US 4,690,911 a zirconia ceramics and a process for producing the same is known, whereas the zirconia ceramic comprises $ZrO_2$, $CeO_2$ and $Al_2O_3$ in the form of tetragonal crystals.

Document DE 195 40 452 Al also describes a zirconium-dioxid based ceramic material comprising 0,5 to 50 vol% $Al_2O_3$.

JP-A-11228221 describes a ceria and titania stabilized zirconia ceramic having alumina particles dispersed therein.

SUMMARY OF THE INVENTION

A primary concern of the present invention is to provide an artificial joint made from a zirconia-alumina composite ceramic, which has the capability of achieving a smooth joint motion with a low friction coefficient, while increasing the wear resistance to reduce an amount generated of wear debris by improving a structure of a zirconia-alumina composite ceramic material.

This object is solved by an artificial joint according to claim 1, claims 2 to 4 are preferred embodiments of the inventive artificial joint according to claim 1.

That is, the artificial joint of the present invention comprises a first bone member and a second bone member, which is slidably engaged to a part of said first bone member to form a joint portion. At least one of the first and second bone members is made from the zirconia-alumina composite ceramic. This zirconia-alumina composite ceramic comprises a matrix phase of zirconia grains and a second phase of alumina grains dispersed in the matrix phase. The zirconia grains of the matrix phase contain ceria as a stabilizer in such an amount that the matrix phase is largely composed of tetragonal zirconia. The zirconia-alumina composite ceramic of the present invention is characterized in that an average grain size thereof is within a range of 0.1 to 0.35 $\mu$m.

In the artificial joint described above, both of the first and second bone members are made from the composite ceramic, and the joint portion is formed by a sliding contact between the composite ceramics. In this case, when the artificial joint is used in vivo for a long time period, it is particularly effective to reduce the amount generated of wear debris and prevent the phenomenon of Osteolysis.

It is preferred that a content of the second phase in the ceramic composite described above is within a range of 25 to 40 vol%.

Moreover, in the artificial joint described above, it is preferred that parts of the alumina grains are dispersed in the interior of the zirconia grains.

Additional features and effects brought thereby of the present invention will be more apparently understood from the best mode for carrying out the invention explained below in detail, referring to the attached drawings.

BRIEF EXPLANATION OF THE DRAWINGS

FIG. 1 is an exploded perspective view of an artificial joint of a zirconia-alumina composite ceramic according to an example of the present invention;
FIG. 2 is a SEM photograph showing a structure of the zirconia-alumina composite ceramic; and
FIG. 3 is a SEM photograph (High Magnification) showing a structure of the zirconia-alumina composite ceramic.

BEST MODE FOR CARRYING OUT THE INVENTION

An artificial joint made from a zirconia-alumina composite ceramic of the present invention is explained in detail below.

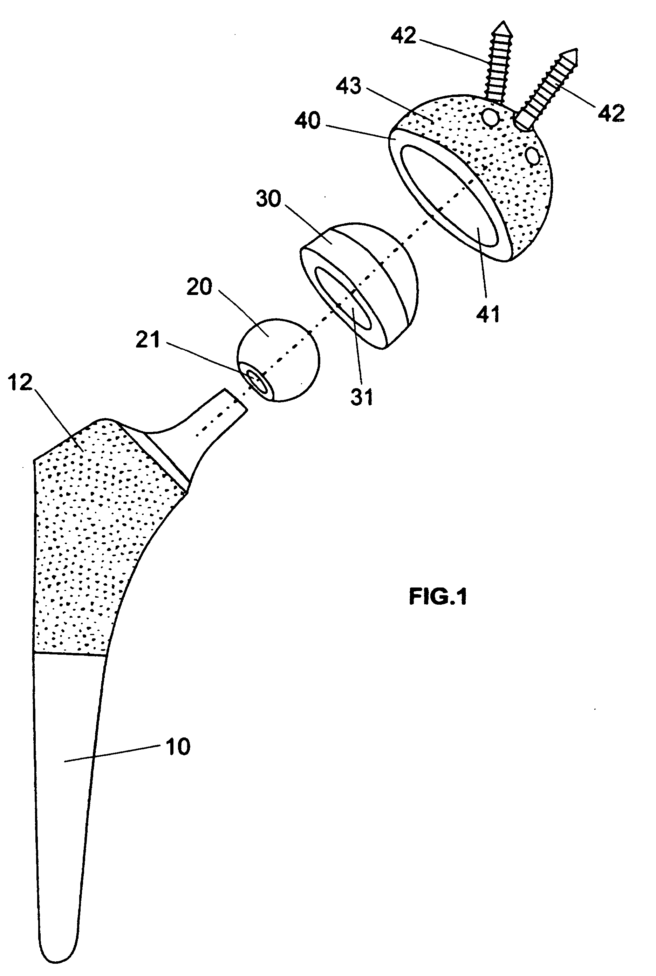

An embodiment of the artificial joint of the present invention is shown in FIG. 1. This artificial joint comprises a caput **20** (first bone member) of a spherical shape, and a stem **10**, one end of which can be inserted into an opening **21** formed at a lower portion of the caput **2** to hold the caput, an inner cup **30** (second bone member) having a first concave **31** having a mirror-polished surface as an inner surface, screws **42** projecting on the outer surface, which can be used to secure the artificial joint in vivo, and an outer cup **40** having a second concave **41,** in which the inner cup **30** can be housed. The outer surface of the caput that is the mirror-polished surface is slidably engaged to the first concave **31** of the inner cup **30** to form a joint portion. In FIG. 1, the numerals **12** and **43** designate porous portions formed in the outer surface of the stem **10** and the outer cup **40**, respectively. The porous portions improve the adhesion between the artificial joint and the bone by an anchor effect.

Both the first bone member such as the caput **20** described above and the second bone member such as the inner cup **30** described above is made from a zirconia-alumina composite ceramic comprising a matrix phase of zirconia grains and a second phase of alumina grains dispersed in the matrix phase.

A content of the matrix phase in the composite ceramic of the present invention is 50 vol% or more. The zirconia grains of the matrix phase is largely composed of tetragonal zirconia. Concretely, an amount of tetragonal zirconia in the matrix phase is 90 vol% or more, and more preferably 95 vol% or more. When this condition is satisfied, it is possible to improve the strength of the composite ceramic according to a stress-induced phase transformation from tetragonal crystal to monoclinic crystal.

To provide the matrix phase that is largely composed of tetragonal zirconia, the zirconia grains contain ceria as a stabilizer. In particular, to obtain the above-described amount of tetragonal zirconia, the content of ceria is within a range of 8 to 12 mol% and more preferably 10 to 12 mol% with respect to the total amount of zirconia. When the content is less than 8 mol%, a sufficient amount of the tetragonal crystal that is a metastable phase is not obtained. As a result, since a ratio of the monoclinic crystal relative to the tetragonal crystal increases, an effect of improving the mechanical strength brought by the stress-induced phase transformation may decrease. On the other hand, when the content is more than 12 mol%, a cubic crystal that is a stable phase at high temperature begins to appear. As a result, since a ratio of the cubic crystal relative to the tetragonal crystal increases, the effect of the stress-induced phase transformation may decrease as in the case of the monoclinic crystal.

If necessary, the matrix phase may further contain at least one selected from the group consisting of titania, calcia and magnesia.

Since titania works as the stabilizer for tetragonal zirconia, a critical stress value required to cause the stress-induced phase transformation increases. As a result, it contributes to further improve the strength of the composite ceramic. In addition, titania also has an effect of accelerating the grain growth of the zirconia grains. Therefore, greater amounts of the alumina grains can be dispersed in the interior of the zirconia grains, as described later. This is effective to further increase the strength of the composite ceramic.

To obtain the above effects, for example, the content of titania is within a range of 0.02 to 4 mol% and particularly 0.05 to 1 mol% with respect to the total amount of zirconia. When the content of titania is less than 0.02 mol%, a sufficient effect brought by the grain growth of zirconia may not be obtained. On the other hand, when the content of titania is more than 4 mol%, abnormal grain growth of the zirconia grains easily occurs. As a result, since the mechanical strength and the wear resistance of the composite ceramic lower, there is a fear that a stable joint motion can not be provided in vivo over a long time period. Therefore, it is required to particularly pay attention to an upper limit of the titania content.

Magnesia and calcia work as the stabilizer for tetragonal zirconia, and contribute to improvements in strength and toughness of the composite ceramic. In addition, since a harmonization of grain boundary between the zirconia grains of the matrix phase and the alumina grains of the second phase is improved, there is an advantage of increasing the grain-boundary strength of the composite ceramic.

To obtain the above-described effects, for example, it is preferred that the content of at least one of magnesia and calcia is within a range of 0.005 to 0.1 mol%, and particularly 0.05 to 0.1 mol%. When the content is less than 0.005 mol%, the effects of the stabilizer may be not sufficiently obtained. On the other hand, when the content is 0.1 mol% or more, there is a fear of inhibiting the stabilization of tetragonal zirconia. In particular, when magnesia is used, elongate crystals of a complex oxide including magnesium appear as a third phase, so that the strength may be reduced by the abnormal grain growth of the elongate crystals. Therefore, it is required to particularly pay attention to an upper limit of the additive amount of magnesia.

The alumina grains of the second phase are dispersed in the grain boundary and/or in the interior of the zirconia grains of the matrix phase. In particular, when the alumina grains are dispersed in the interior of the zirconia grains, the zirconia grains are reinforced to improve the strength of the composite ceramic. This effect is sufficiently demonstrated when a ratio of the number of the alumina grains in the interior of the zirconia grains relative to the entire alumina grains in the composite ceramic is 2 % or more.

It is preferred that a content of the second phase in the composite ceramic is 0.5 vol% or more and 50 vol% or less, and particularly 25 to 40 vol%. When the alumina content is less than 0.5 vol%, there is a fear that the effects of improving the wear resistance of the composite ceramic and increasing the strength of the zirconia grains are not sufficiently obtained. On the other hand, when the content exceeds 40 vol%, there is a tendency that the alumina grains are sintered each other. As a result, variations in strength of the composite ceramic may become larger. In addition, when the content exceeds 50 vol%, the matrix phase of the composite ceramic is composed of the alumina grains. In such a case, the artificial joint with a high degree of reliability of the present invention can not be obtained because of a considerable decrease in toughness.

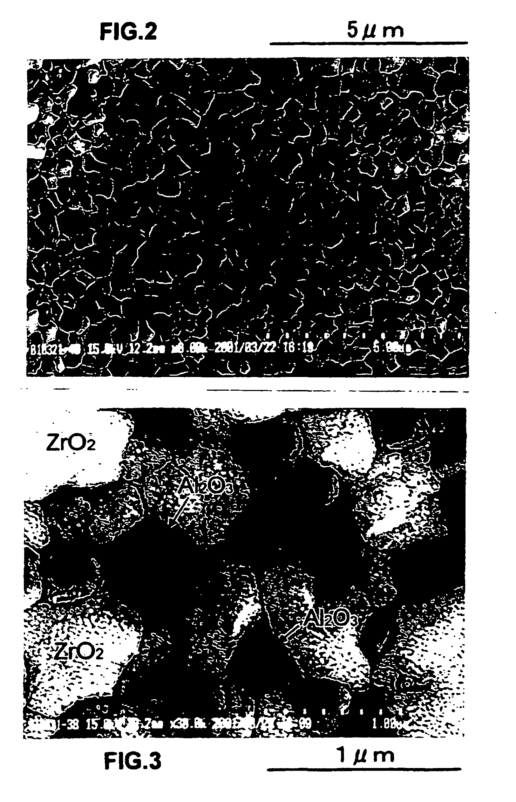

To apply the composite ceramic of the present invention, in which the second phase of the alumina grains and the matrix phase of the zirconia grains are essential components, to artificial joints, the inventors have found that there are very important factors other than the above-described compositions. That is, as shown in FIGS. 2 and 3, the zirconia-alumina composite ceramic of the present invention has a structure, in which extremely fine zirconia and alumina crystal grains are dispersed uniformly. Specifically, an average grain size of the zirconia-alumina composite ceramic for the artificial joints is within a range of 0.1 to 0.35 μm.

When the first bone member is slidably engaged to the second bone member at the joint portion of the artificial joint, a process of polishing the sliding surfaces of the above composite ceramic to obtain super mirror surfaces (e.g., a surface roughness of 0.005 μm Ra or less) is needed to achieve smooth joint motion and function. When the average grain size exceeds 1 μm, it becomes difficult to obtain the super mirror surfaces because the zirconia grains or the alumina grains are easily dropped. In addition, when those grains are dropped from the artificial joint being used in vivo over the long time period, a phenomenon of rapidly reducing the wear resistance occurs. When the average grain size is 0.8 μm or less, the dropping of those grains at the sliding surfaces of the artificial joint that are the super mirror surfaces remarkably reduces. Therefore, it is possible to provide a composite ceramic with a high degree of reliability as a material for artificial joints having stable mechanical properties. When the average grain size is less than 0.1 μm, it becomes difficult to handle raw-material powders in the production process and also sufficiently density the composite ceramic.

Next, an embodiment of the method of producing the artificial joint of the zirconia-alumina composite ceramic of the present invention is explained. First, as a starting material, a mixed powder of zirconia and alumina particles is prepared so as to satisfy the compounding amounts described above. If necessary, titania, calcia and/or magnesia may be added within the above compounding amounts. There is no limitation on production history of the starting material and the grinding and mixing conditions. However, it is required to control the average grain size of the mixed powder such that a sintered body obtained by sintering the mixed powder under adequate conditions has the above-described average grain size.

After the mixed powder is molded by means of uniaxial pressing and/or isostatic pressing to obtain a compact having a required shape, pressureless sintering for the compact is performed at a temperature of 1400 °C or more and 1600 °C or less under atmospheric pressure. The reason for using the temperature 1400 °C or more is because it takes a very long sintering time to obtain a dense sintered body at a temperature less than 1400 °C, or there is a fear that the dense sintered body can not be finally obtained. In addition, since the grain growth easily occurs at the temperature of 1600 °C or more, it becomes difficult to control the sintering process while maintaining the average grain size of less than 1 μm. In particular, there is a tendency that abnormal grain growth of the zirconia grains locally occurs. The obtained sintered body is machined into a desired shape, and the sliding surfaces of the joint portion of the artificial joint are mirror-polished. Thus, the artificial joint of the present invention can be obtained.

By the way, the preparation of the raw-material powders and the control of the sintering temperature are very important to obtain the composite ceramic having an average grain size within a range of 0.1 to 0.35 μm. In consideration of moldability of the mixed powder, it is recommended to use the mixed powder of the zirconia particles having a specific surface area of about 15 m$^2$/g and the alumina particles having an average grain size of about 0.2 μm.

On the other hand, even when a sintering temperature within the range of 1500 °C to 1600 °C is selected, the composite ceramic having the average grain size of 1 μm or less can be obtained. However, when it is desired to produce the composite ceramic having a fine average grain size within the range of 0.1 μm to 0.35 μm, the sintering temperature is selected from the range of 1400 °C or more and 1500 °C or less. Thus, by adopting the adequate combination of the preparation of the raw-material powders and the control of the sintering temperature, it is possible to stably supply the composite ceramic having an ultra-fine structure.

In the artificial joint of the present invention, at least one of the first and second bone members is made from the composite ceramic described above. For example, when one of the first and second bone members is made from the composite ceramic of the present invention, the other one can be made from an ultra-high molecular weight polyethylene or an oxide ceramic mainly comprising alumina. When the combination of the composite ceramic and polyethylene is adopted, the joint portion of the artificial joint is formed by a sliding contact between the composite ceramic and polyethylene. Similarly, when the combination of the composite ceramic and the oxide ceramic is adopted, the joint portion of the artificial joint is formed by a sliding contact between the composite ceramic and the oxide ceramic.

When both of the first and second bone members are made from the composite ceramic of the present invention, an amount of wear caused at the joint portion by using the artificial joint in vivo over a long time period is very small, as compared with the case of using polyethylene. Therefore, it is possible to avoid the problem of Osteolysis. Moreover, since the composite ceramic demonstrates high strength and high toughness, there is an advantage that the reliability is further improved, as compared with the case of using the oxide ceramic mainly comprising alumina. Therefore, it is expected to use the artificial joint with the first and second bone members of the composite ceramic of the present invention as artificial knee, shoulder and cubital joints that are used under more severe sliding conditions in vivo, other than the artificial hip joint. In addition, since the artificial joint of the zirconia-alumina composite ceramic of the present invention has excellent mechanical strength and wear resistance, the life duration can be much extended. Therefore, it is possible to avoid a medical operation of replacing the used artificial joint by a new one after about 10 to 15 years from the previous operation.

EXAMPLES

(Example 1 and Comparative Examples 1-3)

A tetragonal zirconia powder (tetragonal zirconia content: 98 vol%) containing 10 mol% of ceria, 0.05 mol% of titania and 0.05 mol% of calcia with respect to the total amount of zirconia was mixed with 30 vol% of an alumina powder with respect to the entire volume of a zirconia-alumina composite ceramic to obtain a mixed powder. After this mixed powder was molded by means of uniaxial pressing and isostatic pressing to obtain a compact having a required shape, pressureless sintering for this compact was performed at 1400°C for 5 hours under atmospheric pressure. As a result, the zirconia-alumina composite ceramic of Example 1 having the average grain size of 0.22 μm was obtained. To evaluate the wear resistance and the friction coefficient of this composite ceramic, a pin-on-disc wear test was carried out in the presence of distilled water as a lubricant.

As comparative examples, an alumina sintered body having the average grain size of 1.43 μm was used as the Comparative Example 1. In addition, a tetragonal zirconia sintered body having the average grain size of 0.19 μm and containing 3 mol% of yttria as the stabilizer with respect to the entire amount of zirconia was used as the Comparative Example 2. Moreover, a zirconia-alumina composite ceramic having the average grain size of 1.38 μm, which

was obtained by pressureless sintering a mixed powder having the same composition as Example 1 at 1530 °C for 5 hours, was used as the Comparative Example 3. The average grain size was measured by the intercept method with use of a scanning electron microscope without making a distinction between zirconia and alumina after a heat treatment was performed to a polished surface of the obtained sintered body.

Next, a shape of the test specimen for the pin-on-disc test is explained. The pin is a cylinder solid having a diameter of 5 mm and a length of 15 mm, and a circular cone having an apical angle of 30° is provided on a top of the cylinder solid. The top end of the circular cone is formed with a flat mirror area having a diameter of 1.5 mm, which is used as a sliding surface. A surface roughness of this sliding surface is 0.005 µm Ra or less. On the other hand, a disc has a diameter of 50 mm and a thickness of 8 mm. A sliding surface of the disc to be made contact with the pin is a mirror polished surface having a surface roughness of 0.005 µm Ra or less. The pin and the disc are made from a same kind of ceramic material. For example, in Example 1, the pin and the disc, which are made from the composite ceramic of the present invention, were used to perform the pin-on-disc test.

After the pin was placed on a circumference having a radius of 22 mm from the disc center on the disc, the pin-on-disc test was performed at different disc rotational speeds (60 mm/sec, 120 mm/sec) and under different loads (20N, 40N, 60N, 80N, 120N), as listed on Table 1. A sliding distance is constant (25 km). Since the diameter of the top end of the pin is 1.5 mm, initial friction pressures on the top end of the pin are 11 MPa(20N), 22MPa(40N), 33MPa (60N), 44MPa(80N), 66MPa(120N), respectively. In each of the test conditions, the test was repeated three times. Therefore, an average value of the three test results was adopted as data. The obtained test results are listed on Table 1.

Table 1

| | Rotational Speed (mm/sec) | Load (N) | Specific Wear Rate $(mm^3/Nm)x10^{-7}$ | Friction Coefficient |
|---|---|---|---|---|
| Example 1 | 60 | 20 | 0.0584 | 0.34 |
| | 60 | 40 | 0.0155 | 0.37 |
| | 60 | 60 | 0.0584 | 0.29 |
| | 60 | 80 | 0.0965 | 0.22 |
| | 120 | 60 | 0.0986 | 0.38 |
| Comparative Example 1 | 60 | 20 | 1.88 | 0.30 |
| | 60 | 40 | 1.35 | 0.38 |
| | 60 | 60 | 1.05 | 0.37 |
| | 60 | 80 | 0.824 | 0.36 |
| | 120 | 60 | 1.92 | 0.45 |
| Comparative Example 2 | 60 | 20 | 0.0997 | 0.27 |
| | 60 | 40 | 0.0419 | 0.28 |
| | 60 | 60 | 0.126 | 0.35 |
| | 60 | 80 | 230 | 0.42 |
| | 120 | 60 | 480 | 0.49 |
| Comparative Example 3 | 60 | 80 | 74.7 | 0.39 |
| | 60 | 120 | 177 | 0.41 |

After a reduction in weight of the pin was measured by use of a gravimeter with a minimum scale of 0.01 mg, a specific ware rate (Wf) was calculated by the following equation.

$$Wf = (W1-W2)/P{\cdot}L{\cdot}\rho$$

Where,

Wf :    Specific wear rate $(mm^3/Nm)$
W1 :    Dry weight (g) of pin before test

W2 : Dry weight (g) of pin after test
P : Load (N)
L : Sliding distance (m)
ρ : Density (g/mm$^3$) of test specimen

After performing ultrasonic cleaning for the pin in distilled water for 10 minutes and then in ethanol for 10 minutes, and then sufficiently drying the pin in a desiccator for 5 days, the dry weight of the pin was measured. In addition, as the density of each of the test specimens, values measured by Archimedes' method in water, i.e., 5.56 x 10$^{-3}$ g/mm$^3$ (Example 1, Comparative Example 3), 6.02 x 10$^{-3}$ g/mm$^3$ (Comparative Example 1), and 3.93 x 10$^{-3}$ g/mm$^3$ (Comparative Example 2), were used.

In addition, the friction coefficient (Cf) was calculated by the following equation.

$$Cf = F/T$$

Where,

F : Friction force (g)
(Value obtained by measuring a load applied to a beam for holding the pin with a stress gauge.)
T : total load (g)
(Total load of the applied load and the beam for holding the pin)

As understood from the results of Table 1, in Example 1, extremely low specific wear rates of less than 1 x 10$^{-8}$ were obtained under all of conditions of the load applied and the rotation speed in the above tests. In addition, the friction coefficient was also small. Therefore, in the case of the sliding contact between the composite ceramics of Example 1, it is possible to obtain an extremely smooth sliding engagement. On the other hand, in the Comparative Example 1, relatively small specific wear rates within a range of 1 x 10$^{-7}$ to 1x10$^{-8}$ were obtained under all of conditions of the load applied and the rotation speed in the above tests. However, those specific wear rates in the Comparative Example 1 are larger than the specific wear rates in Example 1. In addition, in the Comparative Example 2, extremely low specific wear rates were obtained under conditions up to 60 N of the load applied and the rotation speed of 60 mm/sec, which are substantially the same level as Example 1. However, under the condition of 80N of the load applied, considerable wear suddenly happened with a scale of 1 x 10$^{-5}$. Moreover, under conditions of 60N or less of the load applied and the rotation speed of 120 mm/sec, considerable wear happened as in the above case.

Under the test conditions that caused the considerable wear in the Comparative Example 2, the temperature of distilled water as the lubricant increased, and a large amount of water evaporated. Therefore, it is presumed that considerable heat was generated at the sliding surfaces by the friction. In addition, a crystal transformation of the tetragonal crystal to the monoclinic crystal was identified by Raman spectroscopic analysis. Therefore, it is considered that a thermal phase transition based on degradation at low temperature that is peculiar to yttria stabilized zirconia happened. These results show that the composite ceramic of the Comparative Example 2 using yttria as the stabilizer is poor in reliability, and can not be recommended in applications to artificial knee and shoulder joints, which are used under severe sliding conditions to provide the joint motions.

In addition, the zirconia-alumina composite ceramic of the Comparative Example 3 is of the same composition as the Example 1, but different in average grain size from the Example 1. Under the test conditions that the load applied is relatively small, the wear resistance was substantially the same level as the Example 1. However, when the load applied reached 80N or more, considerable wear suddenly happened as in the case of the Comparative Example 2. As a cause of this considerable increase in wear amount, it is believed that since the composite ceramic of the Comparative Example 3 is of the average grain size (=1.38 μm) more than 1 μm, the interfacial strength between the zirconia and alumina grains decreases, so that those grains were easily dropped by the friction. Thus, from comparison of the test results between the Example 1 and the Comparative Example 3, it is possible to understand that the average grain size of the zirconia-alumina composite ceramic of the present invention wields a very large influence to the wear resistance thereof.

(Examples 2-5 and Comparative Example 4)

A tetragonal zirconia powder (tetragonal zirconia content: 98 vol%) containing 10 mol% of ceria and 0.05 mol% of titania with respect to the total amount of zirconia was mixed with 35 vol% of an alumina powder with respect to the entire volume of a zirconia-alumina composite ceramic to obtain a mixed powder. After this mixed powder was molded by means of uniaxial pressing and isostatic pressing to obtain a compact having a required shape, pressureless sintering for the compact was performed at various temperatures shown in Table 2 for a sintering time of 5 hours under atmospheric pressure. As a result, the zirconia-alumina composite ceramics of Examples 2 to 5 and Comparative Example 4 were obtained. In the Comparative Example 4, since the sintering temperature is 1600 °C, an average grain size of the obtained composite ceramic is 1.65 µm, which does not correspond to the composite ceramic of the present invention. The average grain size was measured by the intercept method with use of a scanning electron microscope without making a distinction between zirconia and alumina after a heat treatment was performed to a polished surface of the obtained sintered body.

A pin-on-disc test was performed to the obtained composite ceramics according to the similar manner to the Example 1, and then the specific wear rate and the friction coefficient were calculated. The rotation speed of the disc is 60 mm/sec constant. In addition, the load applied is 60N constant. The obtained results are shown in Table 2, together with the average grain size of the composite ceramic.

Table 2

|  | Sintering Temperature (°C) | Average Grain Size (µm) | Specific Wear Rate $(mm^3/Nm)x10^{-7}$ | Friction Coefficient |
|---|---|---|---|---|
| Example 2 | 1400 | 0.20 | 0.0320 | 0.30 |
| Example 3 | 1450 | 0.35 | 0.0525 | 0.33 |
| Example 4 | 1500 | 0.65 | 0.0789 | 0.31 |
| Example 5 | 1550 | 0.98 | 1.53 | 0.38 |
| Comparative Example 4 | 1600 | 1.65 | 35.6 | 0.39 |

As understood from the results of Table 2, the specific wear rate of the composite ceramic highly depends on the average grain size thereof. In the Examples 2 to 3, extremely low specific wear rates were obtained. However, in the Example 5 that the composite ceramic has an average grain size near 1 µm, the specific wear rate increased up to 1.53 x $10^{-7}$. In the comparative Example 4 that the composite ceramic has the average grain size of 1.65 µm, the specific wear rate was more than about twenty times as large as in the Example 5, and the specific wear rate suddenly increased. The wear amount of this Comparative Example 4 is substantially equal to that of a conventional artificial joint with a combination of polyethylene and a ceramic. As a cause of such a deterioration of the wear resistance of the composite ceramic having the average grain size of 1 µm or more, it is believed that a reduction in strength of the interface structure between the zirconia and alumina grains induced a considerable increase in amounts of the grains dropped.

INDUSTRIAL APPLICABILITY

As described above, since at least one of first and second bone members of the artificial joint of the present invention is made from a zirconia-alumina composite ceramic comprising a first phase of zirconia grains containing ceria as a stabilizer, which is largely composed of tetragonal zirconia, and a second phase of alumina grains dispersed therein, and an average grain size of the composite ceramic is within a range of 0.1 to 0.35 µm, the composite ceramic demonstrates increased wear resistance as well as high strength and high toughness. Therefore, it is possible to provide an excellent artificial joint having an extended life duration, and thereby avoid a medical operation of replacing the used artificial joint by a new one after about 10 to 15 years from the previous operation. In particular, since the artificial joint of the present invention can maintain good wear resistance under more severe conditions as compared with the case of using the conventional artificial joint, applications to artificial knee and shoulder joints are also expected.

**Claims**

1.  An artificial joint comprising a first bone member and a second bone member, which is slidably engaged to a part of said first bone member to form a joint portion, both said first and second bone members being made from a zirconia-alumina composite comprising:

    a matrix phase of zirconia grains containing 8-12 mol% of ceria and 0,02 to 4 mol% of titania with respect to the total amount of said zirconia grains, so that said matrix phase is largely composed of tetragonal zirconia; and

a second phase of alumina grains dispersed in said matrix phase;

wherein a content of said second phase in said composite ceramic is within a range of 25 to 40 vol%, and an average grain size of said composite ceramic is within a range of 0.1 to 0,35 µm.

2.  The artificial joint as set forth in claim 1, wherein parts of said alumina grains are dispersed in the interior of said zirconia grains.

3.  The artificial joint as set forth in claim 1, wherein said matrix phase further contains at least one selected from the group consisting of calcia and magnesia.

4.  The artificial joint as set forth in claim 1, wherein said matrix phase contains 10-12 mol% of ceria.


**Patentansprüche**

1.  Ein künstliches Gelenk, das ein erstes Knochenelement und ein zweites Knochenelement umfaßt, welches mit einem Teil des ersten Knochenelementes gleitend in Verbindung steht, so daß ein erster Gelenkbereich gebildet wird, wobei sowohl das erste als auch das zweite Knochenelement aus einer Zirkoniumdioxid-Aluminiumdioxid Verbindung hergestellt ist, die umfaßt:

    eine Matrixphase aus Zirkoniumdioxid Körnern, die 8 bis 12 mol. % Zerdioxid und 0,02 bis 4 mol. % Titandioxid im Verhältnis zur Gesamtmenge der Zirkoniumdioxid Körner umfaßt, so daß die Matrixphase im wesentlichen aus tetragonalem Zirkoniumdioxid besteht; und

    eine zweite Phase aus Aluminiumdioxidkörnern, die in dieser Matrixphase dispergiert sind;

    wobei ein Anteil der zweiten Phase in dieser Verbundkeramik innerhalb eines Bereichs von 25 bis 40 Vol. % liegt, und eine durchschnittliche Korngröße dieser Verbundkeramik innerhalb eines Bereiches von 0,1 bis 0,35 µm liegt.

2.  Künstliches Gelenk nach Anspruch 1, wobei Teile der Aluminiumdioxid Körner im Inneren der Zirkoniumdioxid Körner dispergiert sind.

3.  Künstliches Gelenk nach Anspruch 1, wobei die Matrixphase ferner wenigstens ein Element aus der Gruppe umfaßt, die Kalziumdioxid und Magnesiumdioxid umfassen.

4.  Künstliches Gelenk nach Anspruch 1, wobei die Matrixphase 10 bis 12 Mol. % Zerdioxid umfaßt.


**Revendications**

1.  Articulation artificielle comprenant un premier élément osseux et un second élément osseux, lequel est engagé par glissement dans une partie dudit premier élément osseux pour former une partie d'articulation, lesdits premier et second éléments osseux étant tous deux constitués d'un matériau composite zircone-alumine comprenant :

    une phase matricielle de grains de zircone contenant 8 à 12 % en moles d'oxyde de cérium et 0,02 à 4 % en moles de dioxyde de titane rapportés à la quantité totale desdits grains de zircone, de telle sorte que ladite phase matricielle est largement composée de zircone tétragonale ; et
    une seconde phase de grains d'alumine dispersée dans ladite phase matricielle,

    dans laquelle la teneur de ladite seconde phase dans ladite céramique composite se situe dans une fourchette de 25 à 40 % en moles et la granulométrie moyenne de ladite céramique composite se situe dans une fourchette de 0,1 à 0,35 µm.

2.  Articulation artificielle selon la revendication 1, dans laquelle une partie desdits grains d'alumine est dispersée à l'intérieur desdits grains de zircone.

3.  Articulation artificielle selon la revendication 1, dans laquelle ladite phase matricielle contient en outre l'un au moins choisi dans le groupe constitué par l'oxyde de calcium et l'oxyde de magnésium.

4. Articulation artificielle selon la revendication 1, dans laquelle ladite phase matricielle contient 10 à 12 % en moles d'oxyde de cérium.

**FIG.1**

**FIG.2**                                                    5 $\mu$ m

**FIG.3**                                                    1 $\mu$ m